# EUROPEAN PATENT SPECIFICATION

(11) **EP 4 651 956 B1**
(45) Date of publication and mention of the grant of the patent: **01.04.2026**
(21) Application number: 24820414.1
(22) Date of filing: 05.12.2024
(51) Int. Cl.: A61Q 19/10, A61Q 19/00, A61K 8/19, A61K 8/20, A61K 8/36, A61K 8/365, A61K 8/73, C11D 9/26

(54) **SOAP BAR COMPOSITIONS WITH IMPROVED SKIN BENEFITS**
SEIFENSTÜCKZUSAMMENSETZUNGEN MIT VERBESSERTEN HAUTVORTEILEN
COMPOSITIONS DE PAIN DE SAVON AUX BÉNÉFICES AMÉLIORÉS POUR LA PEAU

(30) Priority: 09.01.2024 EP 24150989
(43) Date of publication of application: 26.11.2025
(73) Proprietor: Unilever IP Holdings B.V., 6708 WH Wageningen (NL); Unilever Global IP Limited, Wirral, Merseyside CH62 4ZD (GB)
(72) Inventor: BANKAR, Pravin, 6708 WH Wageningen (NL); BAPAT, Mohini, Anand, 6708 WH Wageningen (NL); GHATLIA, Naresh, Dhirajlal, 6708 WH Wageningen (NL); MAJUMDAR, Amitabha, 6708 WH Wageningen (NL); MATHAPATHI, Mruthyunjaya, Swamy, 6708 WH Wageningen (NL); SANZGIRI, Vibhav, Ramrao, 6708 WH Wageningen (NL); SETHNA, Simone, 6708 WH Wageningen (NL)
(74) Representative: Unilever Patent Group
(86) International application number: PCT/EP2024/084841
(87) International publication number: WO 2025/149246

(56) References cited:
- WO-A1-2011/080101
- CN-A- 106 675 895
- CN-A- 110 151 570
- GB-A- 2 459 093
- US-A1- 2021 007 958
- US-B2- 6 730 643

## Description

### Field of the invention

The present invention relates to cosmetic compositions designed to improve the appearance and health of skin. The present invention relates to the field of soap compositions which provide even tone, glow, clarity and barrier benefits to the skin. More particularly, it relates to such compositions having complex carbohydrate comprising starch and PPAR activator.

### Background of the invention

Skin is subject to deterioration through dermatological disorders, environmental abuse (wind, air conditioning, central heating) or through the normal aging process (chronoaging) which may be accelerated by exposure of skin to sun (photoaging). In recent years the demand for cosmetic compositions and cosmetic methods for improving the appearance and condition of skin has grown enormously.

Many people are concerned with their skin quality and appearance and want to work on it. Dermatologists are also keen to ensure that cleansing products support the skin and help improve the quality of the skin. Essentially cleansing products should care for the skin and add back to the skin.

Peroxisome proliferator-activated receptors (abbreviated herein to PPAR) are transcription factors that control lipid metabolism. There are three isotypes PPARα, PPARβ/δ and PPARγ, all of which have been localised in the skin according to Riviers et al, in J. Invest. Dermatol. 111, 1116-1121 (1998). A range of specific fatty acids activates these factors, resulting in anti-inflammatory action, to reduce cutaneous irritation responses, and pro-differentiation/anti-proliferation responses to normalise skin metabolism and provide additional skin-care benefits. In US-A-5981586, Pershadsingh teaches that PPAR ligands can reduce proliferation and inflammation in the skin. In PCT application WO-A-98/32444 Elias et al teaches that PPAR ligands can restore/prevent skin barrier dysfunction. In EP-A-888773 Malnoe et al describes the use of the PPAR activating lipid petroselinic acid in the treatment and prevention of inflammation in superficial tissues. Furthermore, in PCT application WO-A-99/47110, Alaluf et al teach the use of petroselinic acid or glycerides thereof to reduce skin irritation in a treatment for skin intended simultaneously to combat ageing and wrinkling, and also to provide skin lightening properties. In EP-A-709084, Laugier et al describes the use of coriander oil, rich in petroselinic acid, in a skin cosmetic composition for the moisturisation of dry skin. In US-A-5260053, Chappell et al describe deodorant formulations containing inter alia coriander oil, to accomplish odour reduction, by reducing the population of both micrococci and diphtheroids and to mask any lingering androsterone compounds.

WO2018113636 (Unilever) discloses a combination of modified GSH block amino acid mix, comprising cystine, glutamate and glycine, with a PPAR activating fatty acid achieves a synergistic increase in skin lightening, thus counter-acting the drawback of cystine's low solubility.

WO2001008653 (Unilever) discloses a topical composition comprising: (a) a first lipid selected from petroselinic acid and/or docosahexaenoic acid and/or derivatives thereof; (b) a second lipid which is an activator of peroxisome proliferator activated receptors sub-type alpha and/or derivatives thereof and/or mixtures thereof; and (c) a dermatologically acceptable vehicle; with the proviso that the first and second lipids are not the same lipid. The compositions are useful as cosmetic anti-ageing **skin** care creams and lotions.

WO2012110276 (Unilever) discloses Leave-on non-solid oil-continuous skin conditioning compositions comprising 12-hydroxy stearic acid. Compositions contain 12HSA, yet have a relatively low viscosity, so are suitable for spreading on the skin, and are stable on storage and structurally reversible through temperature cycling.

CN106675895A relates to to the technical field of products of daily use chemicals, and particularly relates to low-irritation toilet soap and a preparation method thereof. The toilet soap comprises raw materials in percentage by mass as follows: 8 percent -12 percent of olive oil, 5 percent -8 percent of oleic acid, 4 percent -6 percent of linoleic acid, 1.5 percent -2.5 percent of sodium hydroxide, 2 percent -4 percent of potassium carbonate, 10 percent -15 percent of lecithin, 4 percent -6 percent of sorbitan fatty acid ester, 2 percent -3 percent of abietic acid, 3 percent -5 percent of propolis, 0.2 percent -0.4 percent of sodium bicarbonate, 2 percent -4 percent of starch, 0.2 percent -0.4 percent of sodium acetate, 0.3 percent -0.5 percent of essence and 40 percent -50 percent of water. According to the toilet soap disclosed by the invention, pH value is about 7.2-7.6, skin irritation caused by strong basicity of traditional toilet soap is avoided, and besides, the added propolis and starch not only are beneficial to skin sterilization and nourishment, but also facilitate forming of the toilet soap.

Hydroxystearic acid has been known for some time in the cosmetic industry. Whereas complex carbohydrates such as starch have been used in soap bar as a part of structuring system and has not been reported for any specific benefits but mostly as a filler.

Most people consider skin health and appearance as one of the key indicators of their own beauty and health. This is affected by factors like age, hormonal changes, occurrence of acne and exposure to sunlight and pollution.

Therefore, there is always a need for a composition that works to improve the appearance of skin and it is a highly desired consumer demand to include the skin benefit agents in consumables that are already a part of daily cleansing routine and does not require any extra steps towards taking care of the skin.

Therefore a soap composition that caters to the timeless demand of skin improvement is need of the hour that is affordable and works with the skin enhancing it with continued use.

### Summary of the invention

First aspect of the present invention provides a soap bar composition comprising:
i. 20 to 80 wt% total fatty matter;
ii. 0.1 to 5 wt% electrolyte;
iii. 0.001 wt% to 5 wt.% PPAR activator;
iv. 1 to 45 wt% complex carbohydrate comprising starch; and
v. 10 to 50 wt% water,
wherein the PPAR activator is 10-hydroxystearic acid or 12-hydroxystearic acid or combination thereof.

Second aspect of the present invention provides a process for preparing a soap composition of the first aspect, comprising
i. saponifying a fatty matter with an alkali to produce a saponified mass;
ii. adding 1 to 45 wt% complex carbohydrate comprising starch or modified complex carbohydrate comprising starch by weight of the soap bar composition and water to obtain a soap mass; and
iii. extruding the soap mass resulting from step (b) to obtain the soap bar according to the first aspect,
   wherein 0.01 to 5 wt% of PPAR activator by weight of the soap bar composition is added at step (i) or (ii), and wherein the electrolyte is added at step (i) or (ii),
wherein the PPAR activator is 10-hydroxystearic acid or 12-hydroxystearic acid or combination thereof.

Third aspect of the present invention provides use of PPAR activator and a complex carbohydrate comprising starch in soap composition according to the first aspect, for providing glow to the skin as compared to a soap bar composition without PPAR activator and a complex carbohydrate comprising starch.

Fourth aspect of the present invention provides use of PPAR activator and a complex carbohydrate comprising starch in soap composition according to the first aspect, for providing even skin tone as compared to a soap bar composition without PPAR activator and a complex carbohydrate comprising starch,
wherein the PPAR activator is 10-hydroxystearic acid or 12-hydroxystearic acid or combination thereof.

Fifth aspect of the present invention provides use of PPAR activator and a complex carbohydrate comprising starch in soap composition according to the first aspect, for providing skin clarity as compared to a soap bar composition without PPAR activator and a complex carbohydrate comprising starch,
wherein the PPAR activator is 10-hydroxystearic acid or 12-hydroxystearic acid or combination thereof.

Sixth aspect of the present invention provides use of PPAR activator and a complex carbohydrate comprising starch in soap composition according to the first aspect, for skin spot fading as compared to a soap bar composition without PPAR activator and a complex carbohydrate comprising starch,
wherein the PPAR activator is 10-hydroxystearic acid or 12-hydroxystearic acid or combination thereof.

As used herein the term "comprising" encompasses the terms "consisting essentially of" and "consisting of". Where the term "comprising" is used, the listed steps or options need not be exhaustive. Unless otherwise specified, numerical ranges expressed in the format "from x to y" are understood to include x and y. In specifying any range of values or amounts, any particular upper value or amount can be associated with any particular lower value or amount. Except in the examples and comparative experiments, or where otherwise explicitly indicated, all numbers are to be understood as modified by the word "about". All percentages and ratios contained herein are calculated by weight unless otherwise indicated. As used herein, the indefinite article "a" or "an" and its corresponding definite article "the" means at least one, or one or more, unless specified otherwise. The various features of the present invention referred to in individual sections above apply, as appropriate, to other sections *mutatis mutandis.* Consequently, features specified in one section may be combined with features specified in other sections as appropriate. Any section headings are added only for convenience and are not intended to limit the disclosure in any way. The invention is not limited to the embodiments illustrated in the drawings. Accordingly, it should be understood that where features mentioned in the claims are followed by reference numerals, such numerals are included solely for the purpose of enhancing the intelligibility of the claims and are in no way limiting to the scope of the claims.

Throughout the specification unless otherwise specified, wt% means weight % of the total weight of soap composition of the present invention.

Various components of the composition are described in greater detail below.

### Detailed description of the invention

These and other aspects, features and advantages will become apparent to those of ordinary skill in the art from a reading of the following detailed description and the appended claims. For the avoidance of doubt, any feature of one aspect of the present invention may be utilized in any other aspect of the invention. The word "comprising" is intended to mean "including" but not necessarily "consisting of" or "composed of." In other words, the listed steps or options need not be exhaustive. It is noted that the examples given in the description below are intended to clarify the invention and are not intended to limit the invention to those examples per se. Similarly, all percentages are weight/weight percentages unless otherwise indicated. Except in the operating and comparative examples, or where otherwise explicitly indicated, all numbers in this description and claims indicating amounts of material or conditions of reaction, physical properties of materials and/or use are to be understood as modified by the word "about". Numerical ranges expressed in the format "from x to y" are understood to include x and y. When for a specific feature multiple preferred ranges are described in the format "from x to y", it is understood that all ranges combining the different endpoints are also contemplated.

The present invention provides a soap bar composition comprising 20 to 80 wt% total fatty matter, 0.1 to 5 wt% electrolyte, 0.001 wt% to 5 wt% PPAR activator, 1 to 45 wt% complex carbohydrate comprising starch; and 10 to 50 wt% water,
wherein the PPAR activator is 10-hydroxystearic acid or 12-hydroxystearic acid or combination thereof.

PPAR activators have been known for some time in the cosmetic industry. Whereas complex carbohydrate comprising starch has been used in soap bar as a part of structuring system and has not been reported for any specific benefits but mostly as a filler.

The present inventors surprisingly came across a composition that when worked in a given wt% range of ingredients results in beneficial properties of the skin. The level of efficacy (as measured by relevant methodologies) obtained from the combination of these ingredients on skin benefits is higher than either of them in isolation and in fact complex carbohydrate comprising starch is not even known for such benefits. The results of the composition of the present invention showed synergistic beneficial results when the combination of PPAR activator and a complex carbohydrate comprising starch was evaluated for skin benefits like glow, skin clarity and even skin tone. The compositions of the present invention were also found to be mild, gentle, smooth and soft as perceived by a large number of consumers during testing. This is also a beneficial aspect of the present invention that a soap, which is a part of every person's daily basic hygiene routine is able to perform such functions so as to improve the skin tone, skin clarity, spot fading and skin glow. It was highly surprising finding of the inventors of the present invention, that without any specific compositions such as serums, creams, the composition which is a part of every person's daily routine is able to target and solve these skin problems. Therefore, the inventors were able to achieve a formulation which could target many skin problems at once without having to make any extra effort to do so.

### Soap

The present invention relates to a soap composition. By a soap composition is meant a cleansing composition comprising soap which is in the form of a shaped solid, namely in the form of bars. The bars in turn, could have a variety of shapes including rectangular, square, or oval cross section.

The cleaning soap composition is generally a wash off products have sufficient amounts of surfactants included therein that it is used for cleansing the desired topical surface e.g. the whole body, the hair and scalp or the face. It is applied on the topical surface and left thereon only for a few seconds or minutes and washed off thereafter with copious amounts of water.

The present invention provides a soap composition comprising: 20 to 80 wt% total fatty matter, 0.1 to 5 wt% electrolyte, 0.001 wt% to 5 wt% PPAR activator, 1 to 45 wt% complex carbohydrate comprising starch; and 10 to 50 wt% water,
wherein the PPAR activator is 10-hydroxystearic acid or 12-hydroxystearic acid or combination thereof.

The soap composition of the invention is especially useful for personal cleansing. The soap composition is a soap bar. The soap composition of the present invention preferably comprises 18 to 80% total amount of TFM from soap, preferably 20 to 75% and more preferably 20 to 65 wt% TFM from soap. The term soap means salt of fatty acid. Preferably, the soap is soap of C8 to C24 fatty acids.

The cation may be an alkali metal, alkaline earth metal or ammonium ion, preferably alkali metals. Preferably, the cation is selected from sodium or potassium, more preferably sodium. The soap may be saturated or unsaturated. Saturated soaps are preferred over unsaturated soaps for stability. The oil or fatty acids may be of vegetable or animal origin.

The soap composition may be obtained by saponification of oils, fats or fatty acids. The fats or oils generally used to make soap bars may be selected from tallow, tallow stearins, palm oil, palm stearins, soya bean oil, fish oil, castor oil, rice bran oil, sunflower oil, coconut oil, babassu oil, and palm kernel oil. The fatty acids may be from coconut, rice bran, groundnut, tallow, palm, palm kernel, cotton seed or soyabean.

The fatty acid soaps may also be synthetically prepared (e.g. by the oxidation of petroleum or by the hydrogenation of carbon monoxide by the Fischer-Tropsch process). Resin acids, such as those present in tall oil, may also be used. Naphthenic acids may also be used.

The soap composition may additionally comprise synthetic surfactants selected from one or more from the class of anionic, non-ionic, cationic or zwitterionic surfactants, preferably from anionic surfactants. These synthetic surfactants, as per the present invention, are included in less than 8%, preferably less than 4%, more preferably less than 1.5% and sometimes absent from the composition.

The chain length of soaps depends on the fat or oil feedstock which is usually a blend. For purposes of this specification, "oil" and "fat" are used interchangeably, except where context demands otherwise. Longer chain fatty acid soaps (e.g., C₁₆ palmitic or C₁₈ stearic) are typically obtained from tallow and palm oils, and shorter chain soaps (e.g., C₁₂ lauric) may typically be obtained from, for example, coconut oil or palm kernel oil. The fatty acid soaps produced may also be saturated or unsaturated (e.g., oleic acid).

Typically, longer chain fatty acid soaps (e.g., C₁₄ to C₂₂ soaps) especially longer, saturated soaps are insoluble and do not generate enough foam upon use but they can make the foam creamier and more stable. Conversely shorter chain soaps (e.g., C₈ to C₁₂) and unsaturated soaps (e.g., oleic or linoleic acid soap) lather quickly. However, the longer chain soaps (typically saturated, although they may also contain some level of unsaturated such as oleic) are desirable to maintain structure and not dissolve as readily. Unsaturated soaps (e.g., oleic) are soluble and lather quickly, like short-chained soaps, but form a denser, creamier foam, like the longer chained soaps.

Iodine value is an indicator of unsaturation and there are well known methods of measurement of IV. One method is gas chromatography. In this method, methyl esters of the fatty acids are formed and analysed by the chromatographic technique. In addition, there are wet chemical methods of analyses. It is possible to measure iodine value of a fat blend before saponification. Additionally, it is possible to determine the iodine value of a soap (saponified oil or fatty acids) present in a finished good like a bar of soap or noodles of soap.

It is preferred that the soap composition has pH in the range from 8 to 13, when measured in a 8% solution with distilled water at 25°C.

### PPAR Activator

Peroxisome proliferator-activated receptors (abbreviated herein to PPAR) are transcription factors that control lipid metabolism. There are three isotypes PPARα, PPARβ/δ and PPARγ, all of which have been localized in the skin.

The proportion of fatty acid PPAR ligands in the invention is at least the minimum proportion which demonstrates a reduction of irritancy and/or improvement in skin condition, compared with the same composition in the absence of the PPAR ligand. As would be expected, such minimum proportion will not only vary from compound to compound but also will depend on whether the acid is employed in free form or introduced via its precursor. The minimum proportion can be determined by a patch test method described subsequently herein.

Peroxisome proliferator activated receptors are a known family of nuclear hormone receptors having three subtypes, α, β, γ, of varying tissue distribution. Peroxisome proliferator activated receptors sub-type α (hereinafter referred to as PPAR α) are present in skin. The preferred PPAR activator for the composition of the present invention is Lipid Activators of Peroxisome Proliferator Activated Receptors of sub-type α.

The term "activator of peroxisome proliferator activated receptors of sub-type α" or "PPAR α activator" in the present application means a lipid that activates the nuclear receptor PPAR α.

PPAR α activators according to the invention are 10-hydroxystearic acid or 12-hydroxystearic acid or combination thereof.

The present invention comprises PPAR activator, more preferably the PPAR activator is PPAR α activator. The PPAR activator is present in the composition of the present invention in the range of 0.001 wt% to 5 wt%, more preferably 0.008 to 4.5 wt% and most preferably 0.01 wt% to 4 wt% by weight of the soap composition.

### Hydroxystearic acid

Hydroxystearic acids are C18 chain fatty acids with one or more OH groups present along the hydrocarbon chain. Typically 12-HAS and 10-HAS are well known hydroxystearic acid. It is often used as an emollient in skin care products and is also a common soap ingredient due to its surfactant properties.

The present invention comprises 12-hydroxystearic acid and 10-hydroxystearic acid and most preferably 12-hydroxystearic acid. The hydroxystearic acid is present in the soap composition of the present invention in the range of 0.001 wt% to 5 wt%, more preferably 0.008 to 4.5 wt% and most preferably 0.01 wt% to 4 wt% by weight of the soap composition.

### Complex Carbohydrate

The soap composition of the present invention comprises an complex carbohydrate. There are two types of carbohydrates: simple and complex. Simple carbohydrates are made of one or two sugar molecules. Complex carbohydrates include starch and fiber.

The complex carbohydrate is a polysaccharide and comprises starch. Polysaccharides, meanwhile, have a general formula of Cₓ(H₂O)*_{y}* where *x* and *y* are usually large numbers between 200 and 2500. When the repeating units in the polymer backbone are six-carbon monosaccharides, as is often the case, the general formula simplifies to (C₆H₁₀O₅)*ₙ*, where typically 40 ≤ *n* ≤ 3000.

The complex carbohydrate comprising starch in the soap composition ranges from 1 to 45 wt%, more preferably from 3 to 40 wt% and most preferably from 5 to 35wt% by weight of the soap composition.

Starch (a polymer of glucose) is a preferably polysaccharide used as a storage polysaccharide in plants, being found in the form of both amylose and the branched amylopectin. In animals, the structurally similar glucose polymer is the more densely branched glycogen, sometimes called "animal starch".

It is preferred that the starch is at least 20 wt% by weight of complex carbohydrate, preferably at least 30 wt%, 40 wt%, 50 wt%, 60wt%, 70wt%, more preferably at least 80 wt%, furthermore preferably at least 85 wt%, most preferably at least 90 wt% by weight of complex carbohydrate. In a highly preferred aspect starch is at least 95 wt% by weight of complex carbohydrate.

In another aspect of the present invention the complex carbohydrate is starch or modified starch.

Suitable starch materials include natural starch (from corn, wheat, rice, potato, tapioca), pre-gelatinized starch, various physically and chemically modified starch and mixtures thereof. By the term natural starch is meant starch which has not been subject to chemical or physical modification - also known as raw or native starch.

A preferred starch is natural or native starch from maize (corn), cassava, wheat, potato, rice and other natural sources of it. Raw starch with different ratio of amylose and amylopectin: e.g. maize (25% amylose); waxy maize (0%); high amylose maize (70%); potato (23%); rice (16%); sago (27%); cassava (18%); wheat (30%) pea (35% amylose) and others. The raw starch can be used directly or modified during the process of making the liquid personal cleansing composition such that the starch becomes gelatinized.. It is preferred that the starch is not modified, is either partially or ungelantinized or is pregelatinized. It is most preferred to use ungelatinized or pre-gelatinized starch. It is further preferred to use starch that is native and not modified.

Another suitable starch is pre-gelatinized, which is starch that has been gelatinized before it is added as an ingredient in the present liquid personal cleansing compositions. Various forms are available that will gel at different temperatures, e.g., cold water dispersible starch.

It is preferred that when particle size of starch ranges between 2-70µm more preferably 4-50µm and most preferably 5-40µm while particle shapes can be smooth oval, oblate spheroid or polygonal and preferably smooth oval/spheroid. It is most preferred that the native starch of particle size in the range of 2-70µm µm, more preferably 4-50µm, and most preferably 5-40µm is used.

### Electrolyte

Inclusion of small amount of an electrolyte (other than soap) can influence the liquid and solid phase ratio. Increasing the electrolyte content lowers the solubility of soap thereby increasing the solid phase amount, on the other hand lowering the electrolyte levels make the bars softer.

Electrolytes as per this invention include compounds that substantially dissociate into ions in water. Electrolytes as per this invention are not an ionic surfactant. Suitable electrolytes for inclusion in the soap making process are alkali metal salts. Preferred alkali metal salts include sodium sulfate, sodium chloride, sodium acetate, sodium citrate, potassium chloride, potassium sulfate, sodium carbonate and other mono or di or tri salts of alkaline earth metals, more preferred electrolytes are sodium chloride, sodium sulfate, sodium citrate, potassium chloride and especially preferred electrolyte is sodium chloride sodium sulphate, sodium citrate or a combination thereof. For the avoidance of doubt, it is clarified that the electrolyte is a non-soap material. It is preferred that the electrolyte is included in the soap composition during the step of saponification to form the soap.

The composition of the invention comprises 0.1 to 5 wt %, more preferably in the range of 0.5 to 3 wt % electrolyte, and most preferably 0.7 to 2.5% by weight of the composition. Preferred electrolytes include sodium sulfate, sodium chloride, sodium citrate, potassium chloride, potassium sulfate, sodium carbonate and other mono or di or tri salts of alkaline earth metals, more preferred electrolytes are sodium chloride, sodium sulfate, potassium chloride and especially preferred electrolytes are sodium chloride and sodium sulfate and combinations thereof. For the avoidance of doubt is clarified that the electrolyte is a non-soap material.

It is most preferred that sodium sulphate and/or sodium chloride and or sodium citrate or combinations thereof are used as electrolytes for the composition of the present invention.

It is preferred when sodium sulphate is present, it is present in the range of 0.1 to 5 wt %, more preferably in the range of 0.5 to 3 wt % electrolyte, and most preferably 0.7 to 2.5% by weight of the composition. It is preferred that sodium sulphate is at least 0.1 wt%, more preferably at least 0.5wt%, and most preferably at least 0.7 wt% and it is preferred that it is not more than 5wt%, more preferably not more than 3 wt%, furthermore preferably not more than 2.8 wt% and most preferably not more than 2.5wt% of the total weight of composition of the present invention.

It is preferred that when sodium chloride is present, it is in the range of 0.5 to 1.5 wt% of the weight of composition, more preferably 0.7 to 1.3 and most preferably 1.0 to 1.3 wt%. It is preferred that sodium chloride is at least 0.1 wt%, more preferably at least 0.5wt%, and most preferably at least 0.7 wt% and it is preferred that it is not more than 5wt%, more preferably not more than 3 wt%, furthermore preferably not more than 2.8 wt% and most preferably not more than 2.5wt% of the total weight of composition of the present invention.

It is preferred that when sodium citrate is present, it is in the range of 0.5 to 1.5 wt% of the weight of composition, more preferably 0.7 to 1.3 and most preferably 1.0 to 1.3 wt%. It is preferred that sodium citrate is at least 0.1 wt%, more preferably at least 0.5wt%, and most preferably at least 0.7 wt% and it is preferred that it is not more than 5wt%, more preferably not more than 3 wt%, furthermore preferably not more than 2.8 wt% and most preferably not more than 2.5wt% of the total weight of composition of the present invention.

### Water- Moisture content

The soap compositions of the present invention have moisture or water content upto 50wt% of the weight of the soap bar. The soap compositions of the present invention have 10 to 50 wt% of water, preferably 12 to 45 wt%, and most from preferably 15 to 40 wt% by weight of the soap composition.

### Organic and Inorganic Adjuvant Materials

The total level of the adjuvant materials used in the bar composition should be in an amount not higher than 50%, preferably 1 to 50%, more preferably 3 to 45% by wt. of the soap bar composition.

The adjuvant system may optionally include insoluble particles comprising one or a combination of materials. By insoluble particles is meant materials that are present in solid particulate form and suitable for personal washing. Preferably, there are mineral (e.g., inorganic) or organic particles.

The insoluble particles should not be perceived as scratchy or granular and thus should have a particle size less than 300 microns, more preferably less than 100 microns and most preferably less than 50 microns.

Preferred inorganic particulate material includes talc and calcium carbonate. Talc is a magnesium silicate mineral material, with a sheet silicate structure and a composition of Mg₃Si₄(OH)₂₂ and may be available in the hydrated form. It has a plate-like morphology, and is essentially oleophilic/hydrophobic, i.e., it is wetted by oil rather than water.

Calcium carbonate or chalk exists in three crystal forms: calcite, aragonite and vaterite. The natural morphology of calcite is rhombohedral or cuboidal, acicular or dendritic for aragonite and spheroidal for vaterite.

Examples of other optional insoluble inorganic particulate materials include aluminates, phosphates, insoluble sulfates, borates and clays (e.g., kaolin, china clay) and their combinations.

Organic particulate materials include: insoluble polysaccharides such as cellulose; synthetic polymers such as various polymer lattices and suspension polymers; insoluble soaps and mixtures thereof.

Bar compositions preferably comprise 0.1 to 25% by wt. of bar composition, preferably 5 to 15 by wt. of these mineral or organic particles.

An opacifier may be optionally present in the personal care composition. When opacifiers are present, the cleansing bar is generally opaque. Examples of opacifiers include titanium dioxide, zinc oxide. A particularly preferred opacifier that can be employed when an opaque soap composition is desired is ethylene glycol mono- or di-stearate, for example in the form of a 20% solution in sodium lauryl ether sulphate. An alternative opacifying agent is zinc stearate.

The product can take the form of a translucent or transparent soap, in which case it will not contain an opacifier.

It is preferred that the soap composition of the present invention has a pH in the range from 9 to 13, when measured in a 4% solution with distilled water at 25°C.

A preferred bar may additionally include up to 30 wt% benefit agents. Preferred benefit agents include moisturizers, emollients, sunscreens, skin lightening agents and anti-ageing compounds. The agents may be added at an appropriate step during the process of making the bars. Some benefit agents may be introduced as macro domains.

Other optional ingredients like anti-oxidants, perfumes, polymers, chelating agents, colourants, deodorants, dyes, emollients, moisturizers, enzymes, foam boosters, germicides, additional anti-microbials, lathering agents, pearlescers, skin conditioners, stabilisers, superfatting agents, sunscreens may be added in suitable amounts in the process of the invention. Preferably, the ingredients are added after the saponification step. Sodium metabisulphite, ethylene diamine tetra acetic acid (EDTA), borax or ethylene hydroxy diphosphonic acid (EHDP) are preferably added to the formulation.

The composition of the invention could be used to deliver antimicrobial benefits. Antimicrobial agents that are preferably included to deliver this benefits include oligodynamic metals or compounds thereof. Preferred metals are silver, copper, zinc, gold or aluminium. Silver is particularly preferred. In the ionic form it may exist as a salt or any compound in any applicable oxidation state. Preferred silver compounds are silver oxide, silver nitrate, silver acetate, silver sulfate, silver benzoate, silver salicylate, silver carbonate, silver citrate and silver phosphate, with silver oxide, silver sulfate and silver citrate being of particular interest in one or more embodiments. In at least one preferred embodiment the silver compound is silver oxide. Oligodynamic metal or a compound thereof is preferably included in 0.0001 to 2%, preferably 0.001 to 1% by weight of the composition. Alternately an essential oil antimicrobial active may be included in the composition of the invention. Preferred essential oil actives which may be included are terpineol, thymol, carvacol, (E) -2(prop-1-enyl) phenol, 2- propylphenol, 4- pentylphenol, 4-sec-butylphenol, 2-benzyl phenol, eugenol or combinations thereof. Furthermore preferred essential oil actives are terpineol, thymol, carvacrol or thymol, most preferred being terpineol or thymol and ideally a combination of the two. Essential oil actives are preferably included in 0.001 to 1%, preferably 0.01 to 0.5% by weight of the composition.

The soap composition may be made into a bar by a process that first involves saponification of the fat charge with alkali followed by extruding the mixture in a conventional plodder. The plodded mass may then be optionally cut to a desired size and stamped with a desirable indicia. An especially important benefit of the present invention is that, notwithstanding the high amount of water content of the soap bar, compositions thus prepared by extrusion are found to be easy to stamp with a desirable indicia.

The present invention also relates to a process to prepare the soap bar of the invention comprising the step of including substantially all of the structuring system to the soap when it is being produced during the saponification step. Preferably, at least, the polymer is included during the saponification stage.

The invention will now be illustrated by means of the following non-limiting examples.

An expression called total fatty matter is used very widely in the field of soaps and detergents. The term abbreviated to "TFM", is used to denote the wt% of fatty acid and triglyceride residues present in the soap composition without taking into account the accompanying cations. For a soap having 18 carbon atoms, an accompanying sodium cation will generally amount to about 8 wt%. Other cations may be employed as desired, for example zinc, potassium, magnesium, alkyl ammonium and aluminium.

It is preferred that the composition of the invention comprises 18 to 75 wt% TFM, more preferably 20 to 70 wt% TFM and most preferably 20 to 65 wt% by weight of the soap composition.

The term soap means salts of fatty acids in which the accompanying cation may be an alkali metal, alkaline earth metal or ammonium ion, preferably an alkali metal. Preferably, the cation is sodium or potassium. The soap may be saturated or unsaturated and it depends on the nature of the corresponding fatty acid and/or oil used for saponification.

### Form and format

Disclosed is a bar of soap comprising a soap composition of the first aspect of the invention. The bar may be of any shape and size, but preferably is rectangular with rounded edges and of a size that allows it to be held comfortably in one hand.

### Other ingredients

In addition to the saponified fatty matter and the polymeric gel, the bars of soap, preferably comprises one or more of the following other ingredients. Choice of the ingredients and the amounts thereof are largely dependent on the formulation scientists and the purpose for which such bars are made.

### Non-Soap Surfactant

The composition of the invention preferably includes a non-soap surfactant, which acts as a co-surfactant and which is selected from anionic, non-ionic, zwitterionic, amphoteric or cationic surfactant or a combination thereof. Preferably the composition comprises 0.1 to 15 wt % non-soap surfactant. More preferably the composition comprises 2 to 10 wt % non-soap surfactant and most preferably 3 to 6 wt % by weight of the soap composition.

Suitable anionic surfactants include water soluble salts of organic sulphuric reaction products having in the molecular structure an alkyl radical containing from 8 to 22 carbon atoms, and a radical chosen from sulphonic acid or sulphuric acid ester radicals, and mixtures thereof.

Examples of suitable anionic surfactants are sodium and potassium alcohol sulphates, especially those obtained by sulphating the higher alcohols produced by reducing the glycerides of tallow or coconut oil; sodium and potassium alkyl benzene sulphonates such as those in which the alkyl group contains from 9 to 15 carbon atoms; sodium alkyl glyceryl ether sulphates, especially those ethers of the higher alcohols derived from tallow and coconut oil; sodium coconut oil fatty acid monoglyceride sulphates; sodium and potassium salts of sulphuric acid esters of the reaction product of one mole of a higher fatty alcohol and from 1 to 6 moles of ethylene oxide; sodium and potassium salts of alkyl phenol ethylene oxide ether sulphate with from 1 to 8 units of ethylene oxide molecule and in which the alkyl radicals contain from 4 to 14 carbon atoms; the reaction product of fatty acids esterified with isethionic acid and neutralized with sodium hydroxide where, for example, the fatty acids are derived from coconut oil and mixtures thereof.

The preferred water-soluble synthetic anionic surfactants are the alkali metal (such as sodium and potassium) and alkaline earth metal (such as calcium and magnesium) salts of higher alkyl benzene sulphonates and mixtures with olefin sulphonates and higher alkyl sulphates, and the higher fatty acid monoglyceride sulphates.

Suitable nonionic surfactants can be broadly described as compounds produced by the condensation of alkylene oxide groups, which are hydrophilic in nature, with an organic hydrophobic compound which may be aliphatic or alkyl aromatic in nature. The length of the hydrophilic or polyoxyalkylene radical which is condensed with any particular hydrophobic group can be readily adjusted to yield a water-soluble compound having the desired degree of balance between hydrophilic and hydrophobic elements.

Particular examples include the condensation product of aliphatic alcohols having from 8 to 22 carbon atoms in either straight or branched chain configuration with ethylene oxide, such as a coconut oil ethylene oxide condensate having from 2 to 15 moles of ethylene oxide per mole of coconut alcohol; condensates of alkylphenols whose alkyl group contains from 6 to 12 carbon atoms with 5 to 25 moles of ethylene oxide per mole of alkylphenol; condensates of the reaction product of ethylenediamine and propylene oxide with ethylene oxide, the condensate containing from 40 to 80 percent of polyoxyethylene radicals by weight and having a molecular weight of from 5,000 to 11,000; tertiary amine oxides of structure R₃NO, where one group R is an alkyl group of 8 to 18 carbon atoms and the others are each methyl, ethyl or hydroxyethyl groups, for instance dimethyldodecylamine oxide; tertiary phosphine oxides of structure R3PO, where one group R is an alkyl group of from 10 to 18 carbon atoms, and the others are each alkyl or hydroxyalkyl groups of 1 to 3 carbon atoms, for instance dimethyldodecylphosphine oxide; and dialkyl sulphoxides of structure R2SO where the group R is an alkyl group of from 10 to 18 carbon atoms and the other is methyl or ethyl, for instance methyltetradecyl sulphoxide; fatty acid alkylolamides; alkylene oxide condensates of fatty acid alkylolamides and alkyl mercaptans.

Suitable cationic surfactants that can be incorporated are alkyl substituted quarternary ammonium halide salts e.g. bis (hydrogenated tallow) dimethylammonium chlorides, cetyltrimethyl ammonium bromide, benzalkonium chlorides and dodecylmethylpolyoxyethylene ammonium chloride and amine and imidazoline salts for e.g. primary, secondary and tertiary amine hydrochlorides and imidazoline hydrochlorides.

Suitable amphoteric surfactants are derivatives of aliphatic secondary and tertiary amines containing an alkyl group of 8 to 18 carbon atoms and an aliphatic radical substituted by an anionic water-solubilising group, for instance sodium 3-dodecylamino-propionate, sodium 3-dodecylaminopropane sulphonate and sodium N-2-hydroxydodecyl-N-methyltaurate.

Suitable zwitterionic surfactants are derivatives of aliphatic quaternary ammonium, sulphonium and phosphonium compounds having an aliphatic radical of from 8 to 18 carbon atoms and an aliphatic radical substituted by an anionic water-solubilising group, for instance 3-(N-N-dimethyl-N-hexadecylammonium) propane-1-sulphonate betaine, 3-(dodecylmethyl sulphonium) propane-1-sulphonate betaine and 3-(cetylmethylphosphonium) ethane sulphonate betaine.

Further examples of suitable detergent-active compounds are compounds commonly used as surface-active agents given in the well-known textbooks "Surface Active Agents", Volume I by Schwartz and Perry and "Surface Active Agents and Detergents", Volume II by Schwartz, Perry and Berch.

### Opacifier

An opacifier may be optionally present in the composition. When opacifiers are present, the cleansing bar is generally opaque, i.e. "opacification". Examples of opacifiers include titanium dioxide, zinc oxide. A particularly preferred opacifier that can be employed when an opaque rather than a transparent soap composition is desired is ethylene glycol mono- or di-stearate, for example in the form of a 20% solution in sodium lauryl ether sulphate. An alternative opacifying agent is zinc stearate.

### Benefit agents

Preferably the soap composition of the invention comprises one or more benefit agent not already disclosed earlier. Preferably the benefit agent is an emollient, sunscreen, anti-ageing compounds or moisturizers and humectants. The agents may be added at an appropriate step during the process. Some benefit agents may be introduced as macro domains.

Examples of moisturizers and humectants include cetyl alcohol, ethoxylated castor oil, paraffin oils, lanolin and its derivatives. Silicone compounds such as silicone surfactants like DC^{®} 3225C (Dow Corning) and/or silicone emollients, silicone oil (DC-200^{®} ex. Dow Corning) may also be included. Further examples include glycerin, oat kernel flour, Petrolatum, Aquaporin and hydroxyethyl urea.

Sunscreens such as 4-tertiary butyl-4'-methoxy dibenzoylmethane (available under the trade name PARSOL^{®}1789 from Givaudan) or 2-ethyl hexyl methoxy cinnamate (available under the trade name PARSOL^{®} MCX from Givaudan) or other UV-A and UV-B sun-screens may also be added. Further examples include Helioplex^{®} (Diethylhexyl naphthylate), Ensulizole^{®}, Ethylhexyl salicylate, Tinosorb^{®} (S & M), Octocrylene^{®} and Mexoryl^{®}.

Lipids such as cholesterol, ceramides, and pseudoceramides, and exfoliant particles such as polyethylene beads, walnut shells, apricot seeds, flower petals and seeds may also be present.

The composition can also optionally include other ingredients conventionally used in soap such as lather boosters, colourants and opacifiers and skin tone agents such as hexyl resorcinol, Soybean extract (Bowman Birk inhibitor), Octadecenedioic acid, (Arlatone^{®} DC), niacinamide, Seppiwhite^{®}, Acetylglucosamine, Pitera Extract, Symwhite^{®} and Melano-block^{®} (Calcium pantothenate). Further, the composition of the invention may comprise antiaging ingredient such as retinol, hyaluronic acid, Collagen, CoQ10 (ubiquinone), retinyl propionate, peptides, retinyl palmitate, Jasmonic acid derivatives and Proxylane^{®}.

Other adjunct materials may include germicides and preservatives. These ingredients normally will be in amounts less than 2 wt %, usually less than 0.5 wt % and may include silver salts and silver compounds, thymol, terpineol and their analogues, ZPTO, chloroxylenol, PCMX, triclosan and trichlorocarbanilide.

The soap composition may include structurants. These may include water insoluble particulate material. Structurants may, individually or combined, support 0 to 25 wt%. Preferred inorganic particulate material includes talc and calcium carbonate. Talc is a magnesium silicate mineral material, with a sheet silicate structure represented by the chemical formula Mg₃Si₄(O)₁₀(OH)₂ and may be available in the hydrated form. Talc has a plate-like morphology and is substantially oleophilic/hydrophobic.

Examples of other optional insoluble inorganic particulate materials include zeolites aluminates, silicates, phosphates, insoluble sulfates, clays (e.g., kaolin, china clay), titanium oxide, zinc oxide and their combinations.

The compositions of the invention may additionally comprise anti-cracking agents such as acrylate polymers.

The term "slip modifier" is used herein to designate materials that when present at relatively low levels (generally less than 1.5% based on the total weight of the bar composition) will significantly reduce the perceived friction between the wet bar and the skin. The most suitable slip modifiers are useful, individually or combined, at a level of 1% or less, preferably from 0.05 to 1% and more preferably from 0.05 to 0.5%.

The composition of the present invention optionally comprises modified poly ethylene glycol in the range of 0.01 to 0.08% as a slip modifier and/or for other sensory benefits. The composition of the present invention may also optionally comprise modified ethylene acrylate copolymer in the range of 0.1 to 0.05% as a benefit agent.

Suitable slip modifier include petrolatum, waxes, lanolins, poly-alkane, poly-alkene, polyalkyene oxides, high molecular weight polyethylene oxide resins, silicones, polyethylene glycols and mixtures thereof.

Free fatty acids (FFA) up to 3% such as coconut fatty acid, PKO fatty acid, lauric acid are commonly used in soap bars for overall quality and process improvement. Free fatty acid higher than 3% could lead to soft and sticky mass and could negatively impact one or more physical feature. In at least one form, level of FFA in compositions of the invention is 0.05 to 3%, preferably 0.1 to 2%, more preferably 0.1 to 1.5 wt%.

A variety of test method have been used to determine properties of the soap compositions.

The test methods are hardness testing protocol, using a 30° conical probe which penetrates to depth of 15 mm. Another test is the rate of wear (RoW) which relates to the amount of material which is lost by a soap bar product under controlled conditions. These conditions for use, mimic approximately the way consumers use the product. A further test is done to check for the extent of as the physical damage which may result (or not) from the sequence of washdown and drying of the bar. Yet another test is to determine "mush" defined as the jelly, creamy material that forms when toilet soap bars absorb water. The Mush Immersion Test gives a numerical value of the amount of mush formed on a bar.

All the aforesaid test methods have been described in US20190016994 A1 (Unilever).

### Process of the invention

In accordance with a second aspect is disclosed a process for preparing a soap composition according to the first aspect, the process comprising the steps of:
i) saponifying a fatty matter with an alkali to produce a saponified mass;
ii) adding 1 to 45 wt% complex carbohydrate comprising starch or modified complex carbohydrate comprising starch by weight of the soap bar composition and water to obtain a soap mass; and
iii) extruding the soap mass resulting from step (ii) to obtain the soap bar according to the first aspect.
   wherein 0.01 to 5 wt% of PPAR activator by weight of the soap bar composition is added at step (i) or (ii); and
   wherein 0.1 to 5 wt% of an electrolyte by weight of the soap bar composition is added at step (i) or (ii),
wherein the PPAR activator is 10-hydroxystearic acid or 12-hydroxystearic acid or combination thereof.

It is preferred that in the process of the present invention, the electrolyte is selected from the group of sodium carbonate, sodium citrate, sodium sulphate, sodium chloride and mixtures thereof and is preferably sodium sulphate and/ or sodium chloride and most preferably sodium sulphate.

It is preferred that in the process of the present invention, the non-soap surfactant is added at step (i).

In the process of the present invention, the soap composition comprises 20 to 80 wt% total fatty matter and 10 to 50 wt% moisture.

It is preferred that in the process of the present invention, the sodium sulphate is in the range of 0.1 to 2 wt% of total weight of the composition.

It is preferred that in the process of the present invention, the starch is native starch, pre-gelatinized starch, converted starch or a mixture thereof and most preferably in the range of 1 to 45 wt% by weight of the soap bar composition.

The PPAR activator is 10- hydroxystearic acid or 12-hydroxystearic acid or combination thereof.

The electrolyte in step (i) is added in the range of 0.1 to 5 wt% of total weight of the composition.

The present invention provides use of PPAR activator selected from 10-hydroxystearic acid or 12-hydroxystearic acid or combination thereof and a complex carbohydrate comprising starch in soap bars having moisture in the range of 10 to 50wt% for achieving hardness of at least 3 Kg-F measured at 42°C.

The soap bar composition according to the present invention may be produced on a commercial scale by any of the processes known to a person skilled in in the art. The soap could be a cast melt soap bar or an extruded soap bar. Preferably the soap bar composition of the present invention is prepared using the extrusion route.

The specific process that may be employed using the above described general process for soap composition manufacture.

The present invention provides use of PPAR activator selected from 10-hydroxystearic acid or 12-hydroxystearic acid or combination thereof and a complex carbohydrate comprising starch in soap composition according to the first aspect, for providing glow to the skin as compared to a soap bar composition without PPAR activator and a complex carbohydrate comprising starch as measured by various methodologies compared.

The present invention provides use of PPAR activator selected from 10-hydroxystearic acid or 12-hydroxystearic acid or combination thereof and a complex carbohydrate comprising starch in soap composition according to the first aspect, for providing even skin tone as compared to a soap bar composition without PPAR activator and a complex carbohydrate comprising starch as measured by various methodologies compared.

The present invention provides use of PPAR activator selected from 10-hydroxystearic acid or 12-hydroxystearic acid or combination thereof and a complex carbohydrate comprising starch in soap composition according to the first aspect, for providing skin clarity and spot fading to the skin as compared to a soap bar composition without PPAR activator and a complex carbohydrate comprising starch as measured by various methodologies compared.

The present invention provides use of PPAR activator selected from 10-hydroxystearic acid or 12-hydroxystearic acid or combination thereof and a complex carbohydrate comprising starch in soap composition according to the first aspect, for other skin health, aging, barrier and appearance benefits as measured by various methodologies compared to a soap bar composition without PPAR activator and a complex carbohydrate comprising starch. The invention will now be illustrated by means of the following non-limiting examples.

### EXAMPLES

The soap compositions for the examples were prepared in accordance with the present invention.

The soap manufacturing process starts primarily with the basic step of saponification of the fat charge with an alkali to produce a soap mass. The process may or may not include other excipients and additives such as but not limited to electrolytes, polyols. A required quantity of fatty acids & other raw materials were weighed & charged into the mixer. An aqueous solution of alkali was used for this neutralization reaction. The completion of the neutralization was checked through phenolphthalein indicator test and if required additional alkali to be added to ensure completion of the neutralization reaction. In-process quality check to be done before the soap mass is passed through further processes including but not limited to passing of soap mass through chilled rolls and then to refiner to convert into noodle form.

In the final step, the soap noodles are added to the mixer. They are crushed. Requisite

amount of other formula additives including but not limited to starch, 12HSA are added and mixing continued. Required colourants are dissolved in water prior to addition to the mixer contents. Fragrance is added at the end and the mixing is continued to get uniform macro mixing. The resultant formula mass is then dropped & conveyed for further mechanical processes including but not limited to its extrusion from a plodder post which it is stamper into soap bars of desired shape, size and branding.

Soap compositions (E1 and E2) were prepared according to the present invention having starch levels 11 wt% and 17 wt% respectively by weight of the soap bar composition of the present invention, were prepared using the formulation as shown in Table 1 and formulation E3 was outside the scope of the present invention in which starch was absent. All the soap bar compositions had hydroxystearic acid.

The compositions E1 to E3 were evaluated for clinical dermatological assessment, gives mean CFB- change from baseline values and also consumer testing based on the scale of 1 to 5 based on the consumer perception.

### GLOW & RADIANCE - COMPOSITE GLOW SCALE

Glow & Radiance is assessed based on the method as provided in (Kumari R et al. Int J Res Dermatol. 2022 Nov;8(6):593-600). A double blind randomised clinical study was performed under dermatologist supervision and evaluated by a dermatologist. This included panelists from Fitzpatrick scale 3 to 5 and with dark spots as per dermatologist discretion. Panelists were provided a wash off period to acclimatise them and the study duration extended from a minimum of 1 week to a maximum of 12 weeks. Dermatologists used in this study were trained and validated for the evaluation skills. The four visual skin attributes which mainly impact the G&R assessment were identified to be glow, clarity, evenness of skin tone and spot reduction .

Scale and calculations- The "multiparametric composite G&R scale" is based on the visual perception of, glow, clarity, evenness of skin tone and spot reduction. Each parameter is independently assessed between (1-5) with a provision of intermediate scoring system. The scale is inclusive of all skin colors and ethnicities. The sensitivity of the scale has been validated with various skin products on skin of colour for visual representation of the scale on skin of colour population).

Skin Tone Unevenness- The subjects reported for assessment were instructed to wash their face and forearm with water and acclimatized at the testing center. Post acclimatization the subjects are assessed by trained dermatologists using Skin Tone Evenness Scale, which is based on the assessment of skin color variation across the test area. The skin tone evenness is scaled between 1- 5 where lower value indicates uneven skin tone and higher value indicates even skin tone. Skin tone evenness is a result of phenotypic changes that is mostly marginal and difficult to perceive and statistically significant increase in the skin tone evenness score observed post product use is an indication of significant improvement in skin tone evenness.

Skin Clarity - The subjects reported for assessment were instructed to wash their face and forearm with water and acclimatized at the testing center. Post acclimatization the subjects are assessed by trained dermatologists using Skin Clarity Scale, which is based on the reflectance/ luminance assessment. The skin clarity is scaled between 1-5 where lower value indicates unclear skin and higher value indicates clear skin. Skin clarity is a result of phenotypic changes that is mostly marginal and difficult to perceive, Statistically significant increase in the skin clarity score observed post product use is an indication of improvement in skin clarity.

Spot Fading- The subjects reported for assessment were instructed to wash their face and forearm with water and acclimatized at the testing center. Post acclimatization the subjects are assessed by trained dermatologists using Spot Reduction Scale. The spot reduction is scaled between 1- 5 where lower value indicates no change in the spot and higher value indicates perceivable reduction or fading in the spot being assessed. Spot reduction is a result of phenotypic changes that is mostly marginal and difficult to perceive, statistically significant increase in the spot reduction score observed post product use is an indication of spot fading.

### Consumer Trials

Consumers of different diaspora and of different starting skin type were used with different ethnicities. Studies done on more than a 1000 consumers across geographies showed preference for products with the soap compositions E1 to E3 within 1 week of use of the products. This included parameters of glow, soft and smooth skin, Mild and gentle on skin. Analysis was done using industry standard statistical methods. An extract of data from a single market cluster is given in Table 1. The scale was 1 to 5, with 1 meaning disagree strongly and 5 meaning agree strongly based on the following questions:
i. Is the soap mild and gentle on my skin?
ii. Does the soap makes my skin soft and smooth?
iii. Does the soap give a beautiful glow to my skin?

**Table 1:**

| | | **E1** | **E2** | **E3** |
|---|---|---|---|---|
| | | **Inside the scope of the invention** | | **Outside the scope of the invention** |
| **Ingredients** | | | | |
| TFM | | 57 | 51 | 67 |
| Anhydrous Soap | | 60 | 52 | 72 |
| Electrolyte | | 1.4 | 1.7 | 1.4 |
| Starch | | 11.0 | 17 | - |
| Non-soap surfactant | | 2.5 | 2.5 | 1 |
| Water Content | | 18 | 18 | 18 |
| 12-hydroxystearic acid | | 0.01 | 0.01 | 0.01 |
| Minors made upto 100 wt% | | | | |

| **Dermatological Assessment Scale (1 to 5)** (CFB- Mean Change from baseline values) | | | | |
|---|---|---|---|---|
| | i) Composite glow | 0.73 | 1.2 | 0.67 |
| | ii) Evenness of skin tone | 0.28 | 0.34 | 0.01 |
| | iii) Skin clarity and Spot fading | 2.56 | 1.4 | 0.74 |

| **Consumer trials** (Mean score- on the scale (1 to 5) | | | | |
|---|---|---|---|---|
| | i) Gentle and Mild | 4.7 | 4.72 | 4.6 |
| | ii) Soft and Smooth | 4.76 | 4.7 | 4.56 |
| | iii) Glow | 4.69 | 4.68 | 4.55 |

It is clear from the data as presented above that the compositions E1 and E2 prepared according to the present invention show synergy and significantly better results in terms of improved skin benefits such as glow and radiance of skin, evenness of skin tone, skin clarity and spot fading as opposed to E3 where only starch is absent. Further the consumer trial data also showed that the consumers preferred the soap compositions of the present invention namely E1 and E2 than the composition E3 which is a similar composition but out of the scope of the present invention.

## Claims

1. A soap bar composition comprising:
i. 20 to 80 wt% total fatty matter;
ii. 0.1 to 5 wt% electrolyte;
iii. 0.001 wt% to 5 wt% PPAR activator;
iv. 1 to 45 wt% complex carbohydrate comprising starch; and
v. 10 to 50 wt% water;
wherein the PPAR activator is 10- hydroxystearic acid or 12- hydroxystearic acid or combination thereof.

2. A soap composition according to claim 1, wherein the complex carbohydrate comprises at least 50 wt% starch by weight of the complex carbohydrate, wherein the starch is native ungelatinized, pregelatinized or native partially ungelatinized.

3. A soap composition according to claims 1 or 2, wherein the electrolyte comprises a compound that is selected from the group consisting of sodium carbonate, sodium citrate, sodium sulphate, sodium chloride and mixtures thereof.

4. A soap composition according to any one of the preceding claims 1 to 3, wherein the composition has a pH in the range from 9 to 13, when measured in a 4% solution with distilled water at 25°C.

5. A process for preparing a soap composition according to anyone of the preceding claims 1 to 4, the process comprising the steps of:
i) saponifying a fatty matter with an alkali to produce a saponified mass,;
ii) adding 1 to 45 wt% complex carbohydrate comprising starch or modified complex carbohydrate comprising starch by weight of the soap bar composition and water to obtain a soap mass; and
iii) extruding the soap mass resulting from step (ii) to obtain the soap bar composition,
wherein the PPAR activator is added at step (i) or (ii); and
wherein the electrolyte is added at step (i) or (ii);
wherein the PPAR activator is 10- hydroxystearic acid or 12-hydroxystearic acid or combination thereof.

6. A process according to claim 5, wherein the soap composition comprises 20 to 80 wt% total fatty matter and 10 to 50 wt% moisture.

7. A process according to claim 5 or 6, wherein the complex carbohydrate comprises at least 50 wt% starch by weight of the complex carbohydrate, wherein the starch is native ungelatinized, pregelatinized or native partially ungelatinized.

8. A process according to any of the preceding claims from 5 to 7, wherein the electrolyte is selected from the group of sodium carbonate, sodium citrate, sodium sulphate, sodium chloride and mixtures thereof.

9. Use of PPAR activator and a complex carbohydrate comprising starch in soap composition according to anyone of the preceding claims from 1 to 4, for providing glow to the skin as compared to a soap bar composition without PPAR activator and a complex carbohydrate comprising starch.

10. Use of 0.001 wt% to 5 wt% PPAR activator and 1 to 45 wt% of a complex carbohydrate comprising starch, weight percentages based on the weight of the resulting soap composition, in soap composition comprising:
i. 20 to 80 wt% total fatty matter;
ii. 0.1 to 5 wt% electrolyte;
iii. 10 to 50 wt% water;
wherein the PPAR activator is 10- hydroxystearic acid or 12- hydroxystearic acid or combination thereof
for providing even skin tone as compared to said soap bar composition without PPAR activator and a complex carbohydrate comprising starch.

11. Use of 0.001 wt% to 5 wt% PPAR activator and 1 to 45 wt% of a complex carbohydrate comprising starch, weight percentages based on the weight of the resulting soap composition, in soap composition comprising:
i. 20 to 80 wt% total fatty matter;
ii. 0.1 to 5 wt% electrolyte;
iii. 10 to 50 wt% water;
wherein the PPAR activator is 10- hydroxystearic acid or 12- hydroxystearic acid or combination thereof
for providing skin clarity, as compared to a soap bar composition without PPAR activator and a complex carbohydrate comprising starch.

12. Use of 0.001 wt% to 5 wt% PPAR activator and 1 to 45 wt% of a complex carbohydrate comprising starch, weight percentages based on the weight of the resulting soap composition, in soap composition comprising:
i. 20 to 80 wt% total fatty matter;
ii. 0.1 to 5 wt% electrolyte;
iii. 10 to 50 wt% water;
wherein the PPAR activator is 10- hydroxystearic acid or 12- hydroxystearic acid or combination thereof,
for skin spot fading, as compared to a soap bar composition without PPAR activator and a complex carbohydrate comprising starch.

## Patentansprüche

1. Seifenstückzusammensetzung, umfassend:
i. 20 bis 80 Gew.-% Gesamtfettgehalt;
ii. 0,1 bis 5 Gew.-% Elektrolyt;
iii. 0,001 bis 5 Gew.-% PPAR-Aktivator;
iv. 1 bis 45 Gew.-% komplexes Kohlenhydrat, das Stärke umfasst; und
v. 10 bis 50 Gew.-% Wasser;
wobei der PPAR-Aktivator 10-Hydroxystearinsäure oder 12-Hydroxystearinsäure oder eine Kombination davon ist.

2. Seifenkomposition nach Anspruch 1, wobei das komplexe Kohlenhydrat mindestens 50 Ges.-% Stärke, bezogen auf das Gewicht des komplexen Kohlenhydrats, umfasst, wobei die Stärke native ungelatinierte, vorgelatinierte oder native teilweise ungelatinierte Stärke ist.

3. Seifenkomposition nach Anspruch 1 oder 2, wobei der Elektrolyt eine Verbindung umfasst, die aus der Gruppe ausgewählt ist, die aus Natriumcarbonat, Natriumcitrat, Natriumsulfat, Natriumchlorid und Mischungen davon besteht.

4. Seifenzusammensetzung nach irgendeinem der vorhergehenden Ansprüche 1 bis 3, wobei die Zusammensetzung einen pH-Wert im Bereich von 9 bis 13 aufweist, gemessen in einer 4%-igen Lösung mit destilliertem Wasser bei 25°C.

5. Verfahren zur Herstellung einer Seifenzusammensetzung nach irgendeinem der vorhergehenden Ansprüche 1 bis 4, wobei das Verfahren die folgenden Schritte umfasst:
i) Verseifen einer fetthaltigen Substanz mit einer Lauge, um eine verseifte Masse herzustellen;
ii) Zusetzen von 1 bis 45 Gew.-% komplexen Kohlenhydrats, das Stärke umfasst, oder modifizierten komplexen Kohlenhydrats, das Stärke umfasst, bezogen auf das Gewicht der Seifenstückzusammensetzung, und Wasser, um eine Seifenmasse zu erhalten; und
iii) Extrudieren der aus Schritt (ii) resultierenden Seifenmasse, um die Seifenstückzusammensetzung zu erhalten,
wobei der PPAR-Aktivator in Schritt (i) oder (ii) zugegeben wird; und wobei der Elektrolyt in Schritt (i) oder (ii) zugegeben wird;
wobei der PPAR-Aktivator 10-Hydroxystearinsäure oder 12-Hydroxysterainsäure oder eine Kombination davon ist.

6. Verfahren nach Anspruch 5, wobei die Seifenzusammensetzung 20 bis 80 Gew.-% Gesamtfettgehalt und 10 bis 50 Gew.-% Feuchtigkeit umfasst.

7. Verfahren nach Anspruch 5 oder 6, wobei das komplexe Kohlenhydrat mindestens 50 Gew.-% Stärke, bezogen auf das Gewicht des komplexen Kohlenhydrats, umfasst, wobei die Stärke native ungelatinierte, vorgelatinierte oder native teilweise ungelatinierte Stärke ist.

8. Verfahren nach einem der vorhergehenden Ansprüche 5 bis 7, wobei der Elektrolyt aus der Gruppe Natriumcarbonat, Natriumcitrat, Natriumsulfat, Natriumchlorid und Mischungen davon ausgewählt ist.

9. Verwendung eines PPAR-Aktivators und eines komplexen Kohlenhydrats, das Stärke umfasst, in einer Seifenzusammensetzung nach irgendeinem der vorhergehenden Ansprüche 1 bis 4, um der Haut im Vergleich zu einer Seifenstückzusammensetzung ohne PPAR-Aktivator und komplexes Kohlenhydrat, das Stärke umfasst, Glanz zu verleihen.

10. Verwendung von 0,001 Gew.-% bis 5 Gew.-% PPAR-Aktivator und 1 bis 45 Gew.-% komplexen Kohlenhydrats, das Stärke umfasst, wobei die Gewichtsprozente auf das Gewicht der resultierenden Seifenzusammensetzung bezogen sind, in einer Seifenzusammensetzung, die umfasst:
i. 20 bis 80 Gew.-% Gesamtfettgehalt;
ii. 0,1 bis 5 Gew.-% Elektrolyt;
iii. 10 bis 50 Gew.-% Wasser;
wobei der PPAR-Aktivator 10-Hydroxystearinsäure oder 12-Hydroxystearinsäure oder eine Kombination davon ist,
um einen gleichmäßigen Hautton im Vergleich zu der Seifenstückzusammensetzung ohne PPAR-Aktivator und komplexes Kohlenhydrat, das Stärke umfasst, zu erzielen.

11. Verwendung von 0,001 Gew.-% bis 5 Gew.-% PPAR-Aktivator und 1 bis 45 Gew.-% eines komplexen Kohlenhydrats, das Stärke umfasst, wobei die Gewichtsprozente auf das Gewicht der resultierenden Seifenzusammensetzung bezogen sind, in einer Seifenzusammensetzung, die umfasst:
i. 20 bis 80 Gew.-% Gesamtfettgehalt;
ii. 0,1 bis 5 Gew.-% Elektrolyt;
iii. 10 bis 50 Gew.-% Wasser;
wobei der PPAR-Aktivator 10-Hydroxystearinsäure oder 12-Hydroxystearinsäure oder eine Kombination davon ist,
um eine klare Haut im Vergleich zu einer Seifenstückzusammensetzung ohne PPAR-Aktivator und ein komplexes Kohlenhydrat, das Stärke umfasst, zu erzielen.

12. Verwendung von 0,001 Gew.-% bis 5 Gew.-% PPAR-Aktivator und 1 bis 45 Gew.-% eines komplexen Kohlenhydrats, das Stärke umfasst, wobei die Gewichtsprozente auf das Gewicht der resultierenden Seifenzusammensetzung bezogen sind, in einer Seifenzusammensetzung, die umfasst:
i. 20 bis 80 Gew.-% Gesamtfettgehalt;
ii. 0,1 bis 5 Gew.-% Elektrolyt;
iii. 10 bis 50 Gew.-% Wasser;
wobei der PPR-Aktivator 10-Hydroxystearinsäure oder 12-Hydroxystearinsäure oder eine Kombination davon ist,
um ein Verblassen von Hautflecken im Vergleich zu einer Seifenstückzusammensetzung ohne PPAR-Aktivator und komplexes Kohlenhydrat, das Stärke umfasst, zu erzielen.

## Revendications

1. Composition de pain de savon comprenant :
i. 20 à 80 % en poids de matière grasse totale ;
ii. 0,1 à 5 % en poids d'électrolyte ;
iii. 0,001 % en poids à 5 % en poids d'activateur de PPAR ;
iv. 1 à 45 % en poids de complexe d'hydrate de carbone comprenant de l'amidon ; et
v. 10 à 50 % en poids d'eau ;
dans laquelle l'activateur de PPAR est l'acide 10-hydroxystéarique ou l'acide 12-hydroxystéarique ou une combinaison de ceux-ci.

2. Composition de savon selon la revendication 1, dans laquelle le complexe d'hydrate de carbone comprend au moins 50 % en poids d'amidon en poids du complexe d'hydrate de carbone, dans laquelle l'amidon est de l'amidon natif dégélatinisé, prégélatinisé ou natif partiellement dégétalinisé.

3. Composition de savon selon les revendications 1 ou 2, dans laquelle l'électrolyte comprend un composé qui est choisi dans le groupe consistant en carbonate de sodium, citrate de sodium, sulfate de sodium, chlorure de sodium et mélanges de ceux-ci.

4. Composition de savon selon l'une quelconque des revendications précédentes 1 à 3, dans laquelle la composition a un pH dans la plage de 9 à 13, lors de la mesure dans une solution à 4 % avec de l'eau distillée à 25 °C.

5. Procédé de préparation d'une composition de savon selon l'une quelconque des revendications précédentes 1 à 4, le procédé comprenant les étapes de :
i) saponification d'une matière grasse avec un alcalin pour produire une masse saponifiée ;
ii) addition de 1 à 45 % en poids de complexe d'hydrate de carbone comprenant de l'amidon ou de complexe d'hydrate de carbone modifié comprenant de l'amidon en poids de la composition de pain de savon et de l'eau pour obtenir une masse de savon ; et
iii) extruder la masse de savon résultant de l'étape (ii) pour obtenir la composition de pain de savon,
dans lequel l'activateur de PPAR est ajouté à l'étape (i) ou (ii) ; et
dans lequel l'électrolyte est ajouté à l'étape (i) ou (ii) ;
dans lequel l'activateur de PPAR est l'acide 10-hydroxystéarique ou l'acide 12-hydroxystéarique ou une combinaison de ceux-ci.

6. Procédé selon la revendication 5, dans lequel la composition de savon comprend 20 à 80 % en poids de matière grasse totale et 10 à 50 % en poids d'humidité.

7. Procédé selon la revendication 5 ou 6, dans lequel le complexe d'hydrate de carbone comprend au moins 50 % en poids d'amidon en poids du complexe d'hydrate de carbone, dans lequel l'amidon est de l'amidon natif dégélatinisé, prégélatinisé ou natif partiellement dégétalinisé.

8. Procédé selon l'une quelconque des revendications précédentes de 5 à 7, dans lequel l'électrolyte est choisi dans le groupe du carbonate de sodium, du citrate de sodium, du sulfate de sodium, du chlorure de sodium et des mélanges de ceux-ci.

9. Utilisation d'activateur de PPAR et d'un complexe d'hydrate de carbone comprenant de l'amidon dans une composition de savon selon l'une quelconque des revendications précédentes de 1 à 4, pour fournir de la brillance à la peau par rapport à une composition de pain de savon sans activateur de PPAR et complexe d'hydrate de carbone comprenant de l'amidon.

10. Utilisation de 0,001 % en poids à 5 % en poids d'activateur de PPAR et de 1 à 45 % en poids d'un complexe d'hydrate de carbone comprenant de l'amidon, les pourcentages en poids étant basés sur le poids de la composition de savon résultante, dans la composition de savon comprenant :
i. 20 à 80 % en poids de matière grasse totale ;
ii. 0,1 à 5 % en poids d'électrolyte ;
iii. 10 à 50 % en poids d'eau ;
dans laquelle l'activateur de PPAR est l'acide 10-hydroxystéarique ou l'acide 12-hydroxystéarique ou une combinaison de ceux-ci
pour fournir un teint de peau uniforme par rapport à ladite composition de pain de savon sans activateur de PPAR et complexe d'hydrate de carbone comprenant de l'amidon.

11. Utilisation de 0,001 % en poids à 5 % en poids d'activateur de PPAR et de 1 à 45 % en poids d'un complexe d'hydrate de carbone comprenant de l'amidon, les pourcentages en poids étant basés sur le poids de la composition de savon résultante, dans la composition de savon comprenant :
i. 20 à 80 % en poids de matière grasse totale ;
ii. 0,1 à 5 % en poids d'électrolyte ;
iii. 10 à 50 % en poids d'eau ;
dans laquelle l'activateur de PPAR est l'acide 10-hydroxystéarique ou l'acide 12-hydroxystéarique ou une combinaison de ceux-ci
pour fournir une clarté de peau, par rapport à ladite composition de pain de savon sans activateur de PPAR et complexe d'hydrate de carbone comprenant de l'amidon.

12. Utilisation de 0,001 % en poids à 5 % en poids d'activateur de PPAR et de 1 à 45 % en poids d'un complexe d'hydrate de carbone comprenant de l'amidon, les pourcentages en poids étant basés sur le poids de la composition de savon résultante, dans la composition de savon comprenant :
i. 20 à 80 % en poids de matière grasse totale ;
ii. 0,1 à 5 % en poids d'électrolyte ;
iii. 10 à 50 % en poids d'eau ;
dans laquelle l'activateur de PPAR est l'acide 10-hydroxystéarique ou l'acide 12-hydroxystéarique ou une combinaison de ceux-ci
pour décolorer les tâches cutanées, par rapport à ladite composition de pain de savon sans activateur de PPAR et complexe d'hydrate de carbone comprenant de l'amidon.
